# EUROPÄISCHE PATENTSCHRIFT

(11) **EP 2 427 762 B1**
(45) Veröffentlichungstag und Bekanntmachung des Hinweises auf die Patenterteilung: **19.06.2013**
(21) Anmeldenummer: 10716479.0
(22) Anmeldetag: 15.04.2010
(51) Int. Cl.: G01N 33/28, H01F 29/02

(54) **VERFAHREN ZUR GASANALYSE AN LASTSTUFENSCHALTERN**
METHOD FOR GAS ANALYSIS OF ON-LOAD TAP CHANGERS
PROCÉDÉ D'ANALYSE DE GAZ DE GRADUATEURS DE RÉGLAGE EN CHARGE

(30) Priorität: 06.05.2009 DE 102009021036
(43) Veröffentlichungstag der Anmeldung: 14.03.2012
(73) Patentinhaber: Maschinenfabrik Reinhausen GmbH, 93059 Regensburg (DE)
(72) Erfinder: FROTSCHER, Rainer, 93128 Regenstauf (DE); HARTWIG, Ralf, 12683 Berlin (DE)
(86) Internationale Anmeldenummer: PCT/EP2010/002306
(87) Internationale Veröffentlichungsnummer: WO 2010/127759

(56) Entgegenhaltungen:
- WO-A1-2007/115807
- DE-A1- 3 227 840
- JAKOB F ET AL: "Use of gas concentration ratios to interpret LTC & OCB dissolved gas data" PROCEEDINGS OF THE ELECTRICAL INSULATION CONFERENCE AND ELECTRICAL MANUFACTURING AND COIL WINDING CONFERENCE. (COMBINED CONFERENCE). INDINAPOLIS, IN, SEPT. 23 - 25, 2003; [PROCEEDINGS OF THE ELECTRICAL ELECTRONICS INSULATION CONFERENCE AND ELECTRICAL, 23. September 2003 (2003-09-23), Seiten 301-304, XP010670664 ISBN: 978-0-7803-7935-0
- DUVAL M: "The duval triangle for load tap changers, non-mineral oils and low temperature faults in transformers" IEEE ELECTRICAL INSULATION MAGAZINE, IEEE SERVICE CENTER, NEW YORK, NY, US, Bd. 11, Nr. 6, 1. November 2008 (2008-11-01), Seiten 22-29, XP011266847 ISSN: 0883-7554

## Beschreibung

Die Erfindung betrifft ein Verfahren zur quantitativen Analyse von Gasen, insbesondere ein Monitoring-System für Laststufenschalter.

Stufenschalter sind seit vielen Jahren zur unterbrechungslosen Umschaltung zwischen verschiedenen Wicklungsanzapfungen von Stufentransformatoren in großen Zahlen weltweit im Einsatz. Solche Stufenschalter bestehen üblicherweise aus einem Wähler zur leistungslosen Anwahl der jeweiligen Wicklungsanzapfung des Stufentransformators, auf die umgeschaltet werden soll, und einem Lastumschalter zur eigentlichen Umschaltung von der bisherigen auf die neue, vorgewählte Wicklungsanzapfung. Der Lastumschalter weist dazu üblicherweise Schaltkontakte und Widerstandskontakte auf. Die Schaltkontakte dienen dabei zur direkten Verbindung der jeweiligen Wicklungsanzapfung mit der Lastableitung, die Widerstandskontakte zur kurzzeitigen Beschaltung, d. h. Überbrückung mittels eines oder mehrerer Überschaltwiderstände. Die Entwicklungen der letzten Jahre führten jedoch weg von Lastumschaltern mit mechanischen Schaltkontakten. Stattdessen werden vermehrt Vakuumschaltzellen oder auch Thyristoren als Schaltelemente eingesetzt.

Unabhängig vom Umschaltprinzip ist ein Stufenschalter eine mechanisch und elektrisch beanspruchte Vorrichtung, die bei einem technischen Versagen in aller Regel einen, gemessen an ihrem eigentlichen Wert, sehr hohen Folgeschaden im Energieversorgungsnetz verursacht. Folgerichtig liegt es im Interesse von Energieversorgern oder auch Kraftwerksbetreibern, unter Umständen auftretende Schäden am Stufenschalter möglichst frühzeitig zu detektieren und zudem einer festen Fehlerursache zuzuordnen. Bisher ist kein zuverlässiges Verfahren bekannt geworden.

In der DD 218 465 ist ein Verfahren zum Überwachen von ölisolierten Hochspannungsgeräten, insbesondere Öltransformatoren, beschrieben. An Hand dieses Verfahrens soll eine Schadenfrüherkennung mittels der Ermittlung schadensspezifischer Gaskomponenten, z.B. CH4, C2H6, C2 H4, C3H8, C3H6, im Spurenkonzentrationsbereich ermöglicht werden, indem eine kontinuierliche Erfassung mehrerer derartiger Gaskomponenten des an einem Ölkessel im Lösungsgleichgewicht mit dem Isolieröl befindlichen freien Gasen erfolgt.

Dieses Verfahren sei nur beispielhaft genannt und steht repräsentativ für eine ganze Reihe weiterer bekannt gewordener Verfahren, die sich aber ausschließlich mit der Früherkennung von möglichen Schadensfällen an Leistungstransformatoren beschäftigen. Jedoch ist es ausdrücklich nicht möglich, die aus diesen Veröffentlichungen gewonnenen Erkenntnisse von Gas-in-Öl-Analysen oder Dissolved Gas Analysis (DGA) auf ein Monitoringsystem speziell für einen Stufenschalter zu übertragen. In einem Stufenschalter herrschen grundlegend andere Bedingungen als in einem Leistungstransformator, weil ein Stufenschalter Bauteile, wie Überschaltwiderstände und mechanische bewegliche Kontakte, benötigt, die ausschlaggebend für auftretende Störfälle sein können und die ein Leistungstransformator eben nicht benötigt.

Aufgabe der Erfindung ist es demnach, ein Verfahren zur quantitativen Analyse von Gasen als Monitoring-System für Laststufenschalter anzugeben, mit dem frühzeitig zuverlässige Aussagen über sich anbahnende Schäden am Stufenschalter getroffen werden können. Weiterhin ist es eine Aufgabe der Erfindung, diese Aussagen einer bestimmten Fehlerursache zuzuordnen.

Diese Aufgabe wird erfindungsgemäß durch ein Verfahren mit den Verfahrensschritten des ersten Patentanspruches gelöst. Die Unteransprüche betreffen besonders vorteilhafte Weiterbildungen des erfindungsgemäßen Verfahrens.

Die allgemeine erfinderische Idee besteht darin, bestimmte charakteristische Gase, die eine indirekte nachfolgende Zuordnung und ein Maß für Alterungserscheinungen, übermäßige Entladung und/oder Erwärmung am Stufenschalter ermöglichen, auszuwählen, diese charakteristischen Gase, die sich während des Betriebes des Stufenschalters in dessen Isolieröl bilden, in bestimmten Zeitabständen zu messen, aus den gemessenen Werten der definierten Gase aussagekräftige Quotienten zu bilden, die direkte Rückschlüsse auf Alterungserscheinungen, übermäßige Entladung und/oder Erwärmung zulassen, aus einem Abgleich des aktuellen ermittelten Wertes des jeweiligen Quotienten mit dem zuletzt ermittelten Wert des selben Quotienten Tendenzen aufzuzeigen und daraus Warnungen für Alterungserscheinungen, übermäßige Entladung und/oder Erwärmung abzuleiten, wenn sich die entsprechenden Quotienten im Lauf der Zeit oder im Verlauf der absolvierten Schaltungen tendenziell signifikant verändern.

Zwar sind durchaus einzelne Verfahrensschritte der erfinderischen Idee per se bekannt, beispielhaft sei hier auf die Veröffentlichung Jakob F et al: "Use of gas concentration ratios to interpret LCT&OCB dissolved gas data", Proceedings of the electrical insulation conference and electrical manufacturing and coil winding conference, Sept. 23-25, 2003, Seiten 301-304, ISBN:978-0-7803-7935-0, verwiesen. waren zahlreiche Überlegungen und Laborversuche notwendig, um die an sich bekannten Verfahrensschritte derart weiterzuentwickeln, dass ein erfinderisches Verfahren nach den Merkmalen des ersten Patentspruches angegeben werden konnte. Technische Spezifika der Stufenschaltertechnik erforderten in diesem Zusammenhang insbesondere erfinderische Überlegungen bei der Entwicklung aussagekräftiger Quotienten, die nun Rückschlüsse auf übermäßige Alterungserscheinungen, übermäßige Entladung und/oder Erwärmung im Stufenschalter zulassen; dies war bisher nicht möglich. Bei der Erfindung werden danach bestimmte charakteristische Gase, die im Stufenschalter entstehen, zu Quotienten verknüpft, die bestimmten physikalischen Phänomenen zugeordnet werden.

Auf besonders vorteilhafte Weise werden die ermittelten Quotienten in einem zweidimensionalen kartesischen Koordinatensystem dargestellt, in dem die Abszisse durch eine zeitlich messbare Einheit gebildet wird. Auch denkbar ist es, als zeitliche Einheit die fortschreitende Schaltungszahl der vom Stufenschalter vollzogenen Schaltungen zu nehmen. Die aussagekräftigen Quotienten können in dieser Darstellung auf besonders einfache Weise über eine Zeitachse dargestellt werden, wobei sich signifikante Änderungen der entsprechenden Quotienten durch Ausschläge im Diagrammabbild erkennen lassen.

Nach einer nochmals abgewandelten Weiterbildung der Erfindung kann zur Darstellung der Quotienten auch ein dreieckiges Koordinatensystem mit 3 Achsen verwendet werden, wie es beispielsweise bei dem an sich bekannten "Duval-Dreieck" der Fall ist. Bei dieser gewählten Form werden aussagekräftige Quotienten in relativer prozentualer Darstellung zueinander dargestellt.

Das Verfahren soll nachstehend beispielhaft anhand von Zeichnungen noch näher erläutert werden. Es zeigen:
- Figur 1: einen schematischen Ablaufplan eines erfindungsgemäßen Verfahrens
- Figur 2: eine erstes Beispiel einer tendenziellen Auswertung
- Figur 3: ein zweites Beispiel einer tendenziellen Auswertung.

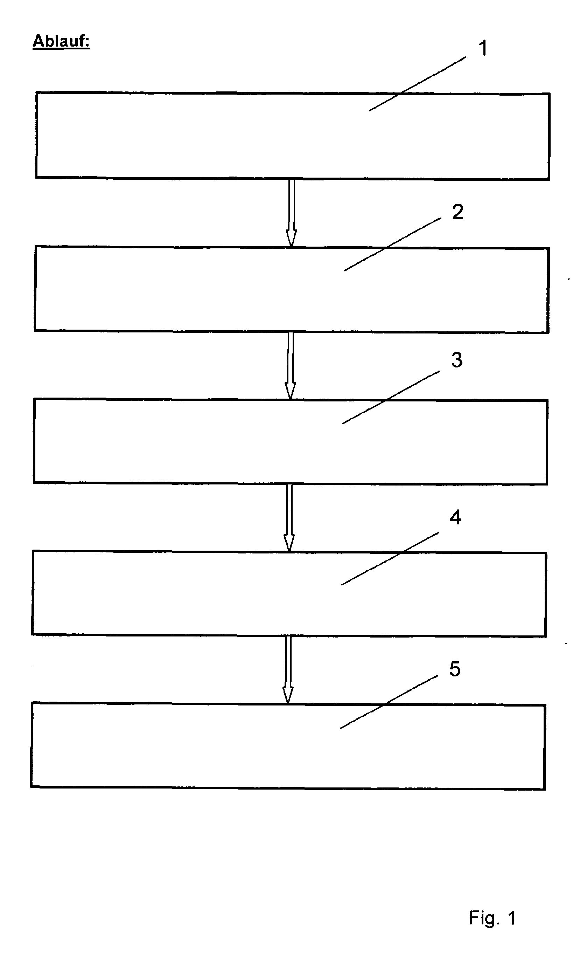

Dem in Fig. 1 schematisch dargestellten Ablaufplan des Verfahrens gemäß der Erfindung zur quantitativen Analyse von Gasen ist zu entnehmen, dass in einem ersten Verfahrensschritt 1 zunächst die für Monitoringzwecke am Stufenschalter geeigneten und damit charakteristischen Gase, die eine nachfolgende Zuordnung und ein Maß für Alterungserscheinungen, übermäßige Entladungen und/oder Erwärmungen ermöglichten, während des Betriebs des Stufenschalters ausgewählt werden. Unter Alterungserscheinung wird in diesem Zusammenhang insbesondere die Ölalterung auf Grund übermäßiger Oxidation des im Stufenschalter verwendeten Isolieröls verstanden.

Für das erfindungsgemäße Verfahren haben sich drei Kategorien von möglichen Fehlergasen als besonders typisch und damit als besonders brauchbar erwiesen. Die charakteristischen Gase lassen sich wie folgt einteilen:

Die erste Kategorie bilden an sich bekannte Lichtbogengase, wie H2 und C2H2, die in Stufenschaltern entstehen, wenn der Lastumschalter von der bisherigen auf die neue, vorgewählte Wicklungsanzapfung, des Stufentransformators umschaltet. Diese Gase entstehen bei allen eingangs erwähnten Umschaltprinzipien, wenngleich in unterschiedlichen Maßen. Die nächste Kategorie der Gase sind Wärmegase, wie CH4, C2H6, C2H4, C3H8 und C3H6, die sich beispielsweise durch die Erhitzung der Überschaltwiderstände des Stufenschalters bilden. Zur dritten und damit letzten Kategorie der auszuwählenden Fehlergase zählen Ölalterungsgase, wie CO und CO2, die dem Grunde nach auch Wärmegase sind, jedoch Rückschlüsse auf den Ölalterungszustand zulassen.

In einem nächsten Verfahrensschritt 2 wird die Konzentration der soeben beschriebenen charakteristischen Gase im Laststufenschalteröl in bestimmten Zeitabständen gemessen. Für das Verfahren typische Zeitabstände sind beispielsweise Messungen zweimal täglich, also morgens und abends. Alternativ zu einer zeitlichen Definition kann sich eine regelmäßige Messung der charakteristischen Gaskonzentrationen auch an der vom Stufenschalter vollzogenen Schaltzahl orientieren. Ein typisches Intervall wäre dann beispielsweise eine Messung nach 20 Schaltungen und jedem weiteren Vielfachen davon.

Nachdem die ermittelten Gaskonzentrationen vorliegen, werden in einem nachfolgenden Verfahrensschritt 3 aussagekräftige Quotienten aus den Selbigen gebildet, die direkte Rückschlüsse auf Alterungserscheinungen, übermäßige Entladungen und/oder Erwärmungen zulassen. Die Quotienten sind:
- CH4/C2H4 für Alterungserscheinungen
- C2H2/C2H6 für Entladungen
- (C2H4+C3H6)/(C2H6+C3H8) für Erwärmung.

Ein nachfolgender Vergleich in einem Verfahrensschritt 4 des aktuell ermittelten Wertes des jeweiligen Quotienten mit dem zuletzt ermittelten Wert desselben Quotienten zeigt eine Tendenz der Messwertentwicklung. Bleibt der Wert des jeweiligen Quotienten in etwa stabil, so ist kein Warnhinweis notwendig und wird demzufolge auch nicht ausgegeben. Ändert sich jedoch einer der oder auch mehrere Quotienten signifikant, insbesondere auch im Vergleich mit mehreren zurückliegenden Werten, also über einen längeren Zeitraum hinweg, oder im Laufe der absolvierten Schaltungen so wird daraus ein dementsprechender Warnhinweis in einem Verfahrensschritt 5 abgeleitet.

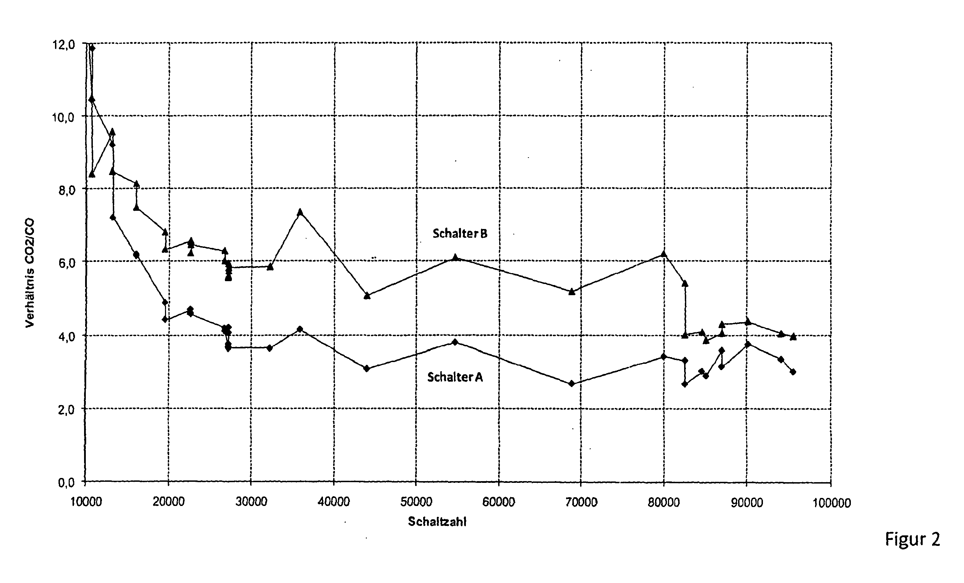

In Figur 2 sind beispielhaft die Verläufe eines weiteren aussagekräftigen Quotienten, nämlich CO2/CO, für zwei untersuchte Stufenschalter über die Anzahl der vollzogenen Schaltungen der Stufenschalter hinweg, dargestellt. An Hand der im zeitlichen Verlauf entstehenden Kurve können damit auf besonders einfache Weise Tendenzen erkannt, analysiert und gegebenenfalls Warnhinweise ausgegeben werden.

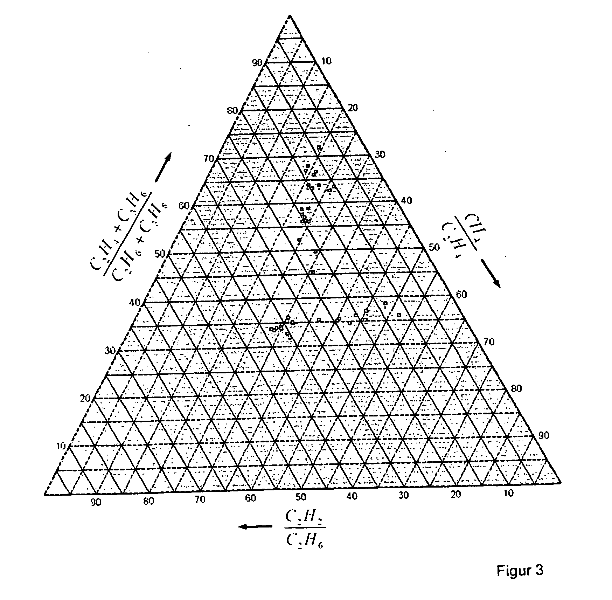

Figur 3 zeigt ein an sich bekanntes dreieckiges Koordinatensystem, wie es beispielsweise als "Duval-Dreieck" zur Anwendung kommt, allerdings in weiterentwickelter und jetzt zu Monitoringzwecken am Stufenschalter geeigneter Form. Jede Seite des gleichseitigen Dreiecks repräsentiert dabei einen aussagekräftigen Quotienten, dargestellt im relativen prozentualen Verhältnis zur Summe der Werte aller Quotienten. Zu einem Punkt in dieser Dreiecksdarstellung gelangt man, indem die prozentualen Einzelwerte der jeweiligen Quotienten zu ein und der selben Messung an den entsprechend zugehörigen Seiten ermittelt und angetragen werden. Jeder angetragene Punkt ist damit durch drei Koordinaten definiert. Eine Tendenzaussage in dieser Darstellungsform ist möglich, indem auf eben beschriebene Weise weitere Punkte zu weiteren Messungen ermittelt und angetragen werden. Bei Änderungen eines oder mehrerer Meßwerte der Gaskonzentrationen ändern sich in Folge die Quotienten und ergeben eine charakteristische Verschiebung der Punkte im Diagramm, die Rückschlüsse auf sich anbahnende Fehler erlaubt.

Mit dem erfinderischen Verfahren ist es damit erstmals möglich, durch Gasanalysen zuverlässige Aussagen über am Stufenschalter auftretende Fehler zu treffen. Weiterhin ist es möglich, über die Beurteilung von Tendenzen einen übermäßigen mechanischen Verschleiß oder sich anbahnende Fehler bereits im Frühstadium zu erkennen.

## Patentansprüche

1. Verfahren zur quantitativen Analyse von Gasen zum Monitoring von ölisolierten Laststufenschaltern,
aufweisend nachfolgende Verfahrensschritte:
- Auswahl während des Betriebes des Laststufenschalters entstehender charakteristischer Lichtbogengase, wie H2 und/oder C2H2, Wärmegase, wie CH4, C2H6 und/oder C2H4, und Ölalterungsgase, wie CO und/oder CO2, die indirekt eine nachfolgende Zuordnung und ein Maß für Alterungserscheinungen, übermäßige Entladungen und/oder Erwärmungen am Stufenschalter ermöglichen
- Messen der Konzentration der charakteristischen Lichtbogengase, Wärmegase und Ölalterungsgase im Öl in bestimmten Zeitabständen
- Bilden aussagekräftiger Quotienten aus den gemessenen Konzentrationen der charakteristischen Lichtbogengase, Wärmegase und Ölalterungsgase, die direkte Rückschlüsse auf Alterungserscheinungen, übermäßige Entladungen und/oder Erwärmungen zulassen
- Aufzeigen einer Tendenz durch Vergleich des aktuell ermittelten Wertes der jeweiligen Quotienten mit einem oder mehrerer zuletzt ermittelten Werten desselben Quotienten
- Ableiten eines Warnhinweises für Alterungserscheinungen, übermäßige Entladungen und/oder Erwärmungen, wenn sich die entsprechenden Quotienten signifikant verändern
**dadurch gekennzeichnet,**
**dass** als zusätzliche charakteristische Wärmegase C3H8 und/oder C3H6 verwendet werden und diese zusätzlichen charakteristischen Wärmegase mit in die Tendenzauswertung einfließen,
**dass** als aussagekräftiger Quotient für die Alterungserscheinungen CH4/C2H4 verwendet wird,
**dass** als aussagekräftiger Quotient für die auftretenden Entladungen C2H2/C2H6 verwendet wird,
und **dass** als aussagekräftiger Quotient für die auftretenden Erwärmungen (C2H4+C3H6)/(C2H6+C3H8) verwendet wird.

2. Verfahren nach Anspruch 1,
**dadurch gekennzeichnet,**
**dass** die aussagekräftigen Quotienten über den Zeitverlauf dargestellt werden.

3. Verfahren nach Anspruch 1 oder 2,
**dadurch gekennzeichnet,**
**dass** die aussagekräftigen Quotienten an Hand der absolvierten Schaltungsanzahl des Stufenschalters dargestellt werden.

4. Verfahren nach einem der Ansprüche 1 bis 3,
**dadurch gekennzeichnet,**
**dass** die aussagekräftigen Quotienten in relativer prozentualer Darstellung in einem dreieckigen Koordinatensystem abgebildet werden.

## Claims

1. Method for quantitative analysis of gases for the monitoring of oil-insulated on-load tap changers, comprising the following method steps:
- selection, during operation of the on-load tap changer, of characteristic arc gases that arise, such as H₂ and/or C₂H₂, hot gases, such CH₄, C₂H₆ and/or C₂H₄, and oil-ageing gases, such as CO and/or CO₂, which indirectly enable a subsequent association with and a measure for ageing phenomena, excessive discharges and/or heating at the tap changer,
- measuring the concentration of the characteristic arc gases, hot gases and oil-ageing gases in the oil at defined intervals in time,
- forming quotients, valid as statements, from the measured concentrations of the characteristic arc gases, hot gases and oil-ageing gases, which allow direct conclusions with respect to ageing phenomena, excessive discharges and/or heating,
- demonstrating a tendency by comparison of the currently determined value of the respective quotient with one or more last-determined values of the same quotient and
- deriving a warning with respect to ageing phenomena, excessive discharges and/or heating when the corresponding quotients significantly change,
**characterised in that**
C₃H₈ and/or C₃H₆ is or are used as additional characteristic hot gases and these additional characteristic hot gases are included in the tendency evaluation,
CH₄/C₂H₄ is used is as quotient valid as a statement for the ageing phenomena,
C₂H₂/C₂H₆ is used as quotient valid as a statement for the discharges which occur and (C₂H₄ + C₃H₆) / (C₂H₆ + C₃h₈) is used as quotient valid as a statement for the heating which occurs.

2. Method according to claim 1, **characterised in that** the quotients valid as statements are illustrated by way of the plot over time.

3. Method according to claim 1 or 2, **characterised in that** the quotients valid as statements are illustrated on the basis of the absoived switching number of the tap changer.

4. Method according to any one of claims 1 to 3, **characterised in that** the quotients valid as statements are imaged in relative percentage illustration in a triangular co-ordinate system.

## Revendications

1. Procédé d'analyse quantitative des gaz pour contrôler des changeurs de prises en charge à isolation par de l'huile comprenant les étapes de procédé suivantes consistant à :
- sélectionner pendant le fonctionnement du changeur de prises en charge, les gaz caractéristiques générés par un arc électrique tel que H2 et/ou C2H2, les gaz chauds tels que CH4, C2H6 et/ou C2H4 et les gaz de vieillissement d'huile tels que CO et/ou CO2 qui permettent indirectement une association suivante et une mesure des phénomènes de vieillissement, de décharge excessive et/ou d'échauffement du changeur de prises,
- mesurer la concentration des gaz d'arc électrique caractéristiques, des gaz chauds et des gaz de vieillissement de l'huile à des intervalles de temps déterminés,
- former des quotients significatifs à partir des concentrations mesurées des gaz d'arc électrique caractéristiques, des gaz chauds et des gaz de vieillissement d'huile permettant de tirer des conclusions directement concernant les phénomènes de vieillissement, les décharges excessives et/ou des échauffements,
- enregistrer une tendance en comparant la valeur déterminée actuellement des quotients respectifs et d'une ou plusieurs valeurs déterminées en dernier lieu pour ces mêmes quotients,
- déduire une indication d'avertissement pour des phénomènes de vieillissement, des décharges excessives et/ou des échauffements si les quotients correspondants varient de manière significative,
procédé **caractérisé en ce que**
- comme gaz chauds caractéristiques supplémentaires, on utilise C3H8 et/ou C3H6 et on introduit ces gaz chauds caractéristiques supplémentaires dans l'exploitation de la tendance,
- on utilise comme coefficient significatif pour les effets de vieillissement CH4/C2H4,
- on utilise comme coefficient significatif pour les décharges produites C2H2/C2H6, et
- on utilise comme quotient significatif pour l'échauffement produit (C2H4+C3H6)/(C2H6+C3H8).

2. Procédé selon la revendication 1,
**caractérisé en ce qu'**
on représente les quotients significatifs en fonction du temps.

3. Procédé selon la revendication 1 ou 2,
**caractérisé en ce qu'**
on représente les quotients significatifs à l'aide du nombre de commutations effectuées par le changeur de prises.

4. Procédé selon l'une des revendications 1 à 3,
**caractérisé en ce qu'**
on représente les coefficients significatifs sous la forme d'une représentation en pourcentages relatifs dans un système de coordonnées triangulaires.
